Europäisches Patentamt

European Patent Office

Office européen des brevets

(19)

(11) Publication number: **0 519 742 A1**

# (12) EUROPEAN PATENT APPLICATION

(21) Application number : **92305661.8**

(22) Date of filing : **19.06.92**

(51) Int. Cl.⁵ : **A61M 15/00**

(30) Priority : **21.06.91 US 719315**

(43) Date of publication of application :
**23.12.92 Bulletin 92/52**

(84) Designated Contracting States :
**DE FR GB IT**

(72) Inventor : **Frola, Frank R.**
**14801 Cavittsville Road**
**Trafford, Pennsylvania 15085 (US)**

(74) Representative : **Hackett, Sean James**
**Marks & Clerk 57-60 Lincoln's Inn Fields**
**London WC2A 3LS (GB)**

(71) Applicant : **DEVILBISS HEALTH CARE, INC.**
**1200 East Main Street, P.O. Box 635**
**Somerset, Pennsylvania 15501 (US)**

(54) **Medical nebulizer control system.**

(57)    An improved medical nebulizer control system. A patient breathes through a nebulizer (10) containing a volume of medicine. A small portion of the patient's respiratory air is diverted to a compact self contained control unit (12) where the patient's respiration is minitored. Upon initialization by depressing a pushbutton (32), a logic control circuit (30) causes a three way valve (20) to connect the output of a small air compressor (37) to the nebulizer (10) as the patient inhales. The flow of compressed air outlet atomizes medicine contained in the nebulizer for inhalation by the patient. A timer in the logic control circuit controls the length of dosage. Upon completion of the dose, the three way valve returns to its original position and the control unit reverts to its respiratory monitoring function.

FIG. I

EP 0 519 742 A1

Technical Field

The invention relates to nebulizers used for respiratory treatment or diagnosis of patients and more particularly to an improved nebulizer control system which utilizes a three way valve to control the air flow that atomizes medicine in the nebulizer.

Background Art

Medical nebulizers are used to atomize medication and mix the resulting aerosol with air prior to inhalation by a patient. Nebulizers are used to treat various respiratory disorders such as emphysema, bronchitis and asthma. They also are used to diagnosis bronchial allergies by introducing a quantity of antigen into the patient's bronchial passages.

Prior to treatment, medicine is placed in the nebulizer. The patient will then inhale and exhale through a mouthpiece attached to an opening in the nebulizer. A second opening in the nebulizer to the surrounding air provides the majority of the air inhaled by the patient and vents the exhalations of the patient. A third opening admits a compressed gas, usually air or oxygen, to the nebulizer to atomize the medicine. Generally, nebulizers use the venturi effect of pressurized gas flowing over a small orifice in the end of a suction tube to draw liquid medicine through the tube and atomize the medicine into very small particles. The atomized medicine mixes inside the nebulizer with the air being inhaled by the patient.

Some form of metering is necessary when the amount of medicine being administered must be controlled. This can be accomplished by cycling the compressed gas on and off to limit the amount of time that atomization occurs to a portion of the patient's inhalation breathing cycle. Thermal sensors have been used to sense when the patient is inhaling and to generate an electrical control signal. The signal activates a control circuit to open a valve to a compressed gas storage tank that provides the atomizing gas. Usually, a treatment start button is pushed to initialize the control circuitry for an atomization cycle. A timer provides a metering function by reclosing the valve to the compressed gas storage tank once the desired dose of medicine has been administered. Such nebulizers tend to be bulky and complex, requiring compressed gas storage tanks and wires connecting the nebulizer to the control unit circuitry in addition to a gas line from the compressed gas storage tank to the control unit. The compressed gas storage tank also can be large and heavy and requires periodic recharging or replacement.

Disclosure of the Invention

The invention is directed to an improved nebulizer control system that uses a three way valve to control gas flow to and from the nebulizer. A control unit includes the three way valve, a small electric air compressor to supply compressed air for atomization, a mass airflow sensor, and nebulizer controls in one compact control unit. Only a single flexible tube connects the control unit to the nebulizer. All elements of the nebulizer system other than the nebulizer and connecting tube are contained in the control unit.

The three way valve selectively connects the nebulizer either to the mass airflow sensor or to the output of the small air compressor. Normally, the three way valve is in a first position where the nebulizer is connected to the mass airflow sensor. As the patient breathes, the air pressure within the nebulizer changes and a portion of the patient's respiratory air will flow through the flexible tube and the three way valve and in or out of the the mass airflow sensor. The mass air flow sensor is electrically connected to logic control circuitry and signals the circuitry whether the patient is inhaling or exhaling.

A pushbutton is provided for a therapist to initialize a treatment cycle. Once the pushbutton has been depressed, the logic control circuitry is responsive to data received from the mass airflow sensor to cause the three way valve to move to a second position when the patient starts to inhale. When the three way valve is in its second position, compressed air from the air compressor is fed to the nebulizer to atomize the medicine for inhalation by the patient. The air compressor will remain connected until a preset time has elapsed, at which time the logic circuitry will cause the valve to return to its original position to reconnect the mass airflow sensor to the nebulizer. The preset time is less than the patient's total inhalation time.

Accordingly, it is an objective of the invention to provide a nebulizer with an associated self-contained control unit that utilizes a three way valve to control air flows.

Other objects and advantages of the invention will be apparent from the following detailed description and the accompanying drawings.

Brief Description of the Drawings

Fig. 1 is a diagram illustrating a nebulizer and associated control unit in accordance with the invention;

Fig. 2 is a schematic diagram of the nebulizer and associated control unit shown in Fig. 1;

Fig. 3 is a diagram illustrating enhancements of the invention; and

Fig. 4 is a schematic diagram of the nebulizer and control unit shown in Fig. 3.

Best Mode for Carrying out the Invention

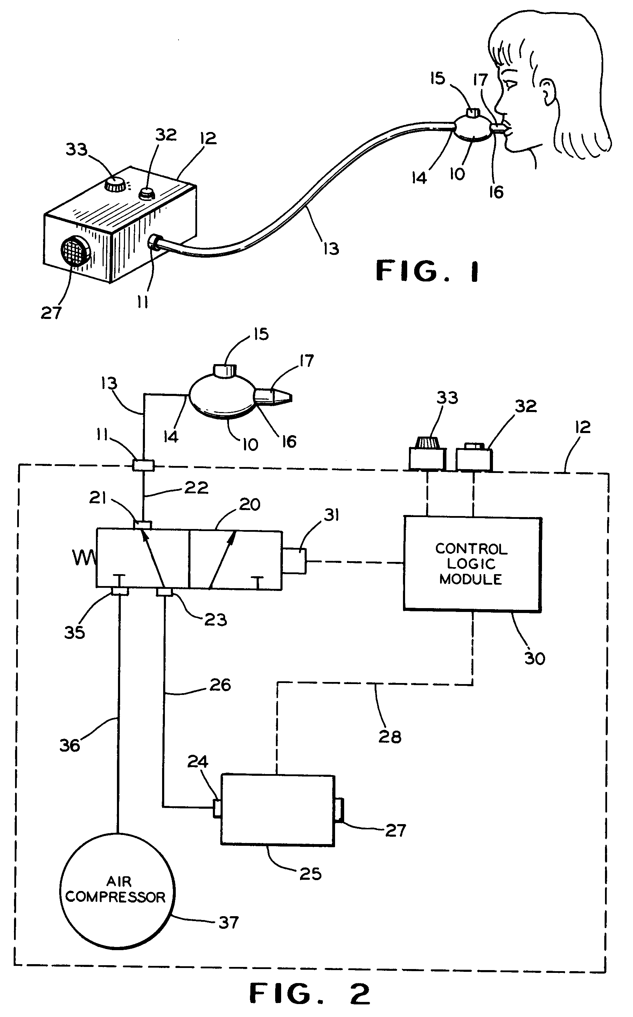

Referring to Figs. 1 and 2 of the drawings, a nebulizer 10 is shown connected to a gas outlet port 11 of a control unit 12 by a single flexible gas tube 13. The nebulizer 10 is of standard design and has three openings. A compressed gas inlet 14 admits compressed gas for atomization of liquid medicine contained in the nebulizer 10. An atmospheric inlet 15 both admits surrounding air to the nebulizer for mixing with the aerosol of medicine for supplying inhalation air to the patient and for discharging air exhaled through the nebulizer 10. Finally, an outlet 16 is connected to a mouthpiece 17 which is placed in the patient's mouth. The patient breathes through the mouthpiece 17 at a normal rate.

The control unit 12 contains all the remaining elements of the system, as illustrated in Fig. 2. Within the control unit 12 is a three way solenoid operated valve 20. The valve 20 has three valve ports. A first valve port 21 is connected to the gas outlet port 11 by an outlet gas line 22. When the three way valve 20 is unenergized, the first valve port 21 is connected to a second valve port 23. The second valve port 23 is connected to an inlet 24 of a mass airflow sensor 25 by an air line 26. The mass airflow sensor 25 is a commercially available unit that generates electric signals in response to gas flowing through it.

As the patient breathes through the nebulizer 10, the primary respiratory air flow is through the atmospheric inlet 15. However, with the three way valve 20 unenergized, the pressure changes within the nebulizer 10 cause a small secondary respiratory air flow through the gas tube 13 to the control unit 12. When the patient exhales, secondary air passes through the three way valve 20 from the first valve port 21 to the second valve port 23, through the line 26, the mass air flow sensor 25 and out an atmospheric vent 27. The secondary air flow reverses when the patient begins to inhale, with a secondary air flow entering the atmospheric vent 27, passing through the mass airflow sensor 25, the line 26, the three way valve 20 and the flexible gas tube 13 to enter the nebulizer 10. The mass air flow sensor 25 senses the direction and beginning of the secondary airflow and transmits electrical signals conveying the information on wires 28 to a control logic module 30.

The control logic module 30 energizes and deenergizes a solenoid 31 to cause movement of the three way valve 20 as will be explained below. A pushbutton 32 is provided for the therapist to initialize the control logic module 30. A dose duration control 33, which may be a dial or switch, is connected to the control logic module 30 to permit the therapist to set the length of time that the solenoid 31 will be energized.

The three way valve 20 has a third port 35 which is connected by a compressed gas line 36 to a supply of compressed gas. For the embodiment illustrated in Fig. 2, a compact electric air compressor 37 is used, but other gases and modes of supply could be substituted, such as a small cylinder of compressed air or oxygen. The use of an air compressor 37 provides a compact self-contained control unit. The air compressor 37 provides pressurized air to the nebulizer 10 to atomize the medicine. The air pressure may vary, for example, from 4 to 20 PSI, with the specific value matched to the particular nebulizer 10 used. Normally, the valve port 35 is closed. When the three way valve 20 is energized, the third valve port 35 is connected to the first valve port 21, allowing pressurized air from the air compressor 37 to pass through the gas tube 13 and into the nebulizer 10, where the pressurized air will pass over a medicine suction tube and produce an aerosol containing medicine.

To use the nebulizer control system, the therapist turns on the air compressor 37, places medicine in the nebulizer 10, and sets the dose duration control 33. The patient then places the nebulizer mouthpiece 17 into his or her mouth and breaths normally through the nebulizer 10. The majority of the patient's respiratory air passes in and out of the nebulizer through the atmospheric inlet 15. The solenoid valve 20 is initially unenergized, so the patient's breathing also causes a small secondary respiratory air flow through the gas tube 13 to the control unit 12. Within the control unit, the unenergized three way valve 20 directs the secondary air through the atmospheric air line 26 and the mass airflow sensor 25 before venting to the atmosphere. The mass airflow sensor 25 provides continuous data to the control logic module 30 concerning the direction of air flow and the beginning of each inhalation and exhalation.

When the therapist is ready to initialize a treatment cycle, he or she presses the pushbutton 32, which signals the control logic module 30 to begin a treatment cycle. Once the pushbutton 32 has been pressed, the next signal from the mass airflow sensor 25 indicating that the patient has started to inhale will cause the control logic module 30 to energize the valve solenoid 31. Energization of the solenoid 31 will cause the three way valve 20 to move to its second position with the third valve port 35 connected to the first valve port 21. Pressurized air now flows from the air compressor 37 through the the three way valve 20 and the gas tube 13 and into the nebulizer 10 where the pressurized air will atomize the medicine. The atomized medicine will mix with the atmospheric air being drawn through the atmospheric inlet 15 and will be inhaled by the patient.

The solenoid 31 will remain energized for the time period preset by the therapist by means of the dose duration control 33. Upon elapse of the time period, the control logic module 30 deenergizes the solenoid 31 to end the treatment cycle. Upon deenergization, the three way valve 20 returns to its first position blocking the supply of pressurized air to the nebulizer and again connecting the first valve port 21 to the second valve port 23. With the return of the three way valve 20 to its original position, the secondary respiratory air flow is reestablished through the mass air flow sensor 25 and the control logic module 30 will be ready for another treatment cycle. The interruption of the pressurized air supply to the nebulizer 10 causes the atomization of medicine to cease. Thus, the amount of medicine administered to the patient can be precisely controlled.

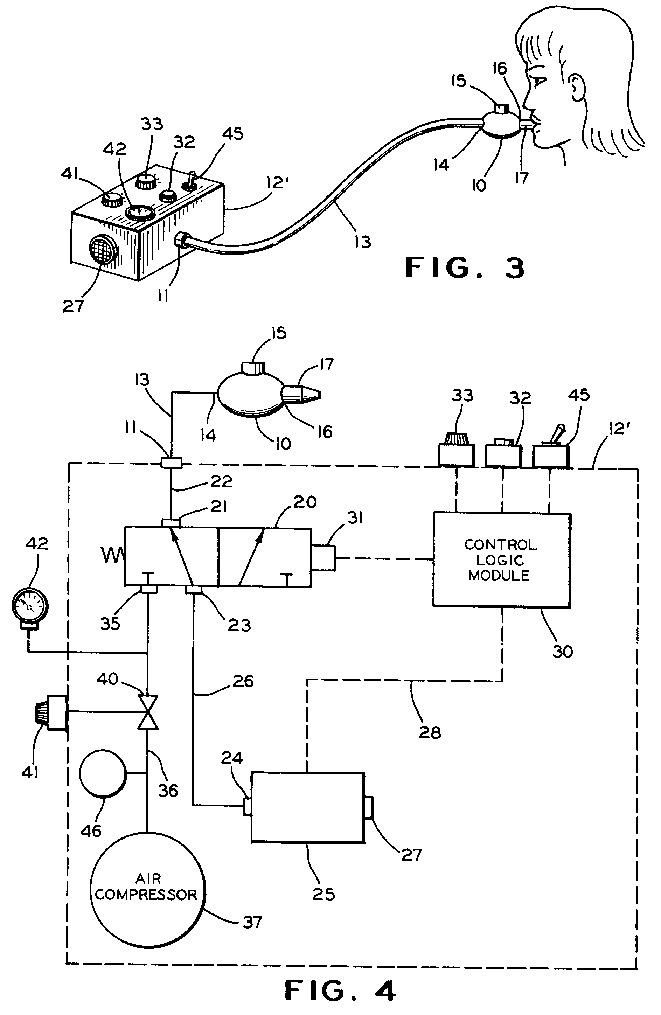

It will be appreciated that there are other embodiments of the invention, one of which is illustrated in Figs. 3 and 4. For example, a pressure regulator 40 may be inserted into the compressed gas line 36 connecting the air compressor 37 to the third valve port 35 of the three way valve 20. The pressure regulator 40 is adjustable by the therapist with a dial 41 mounted on the exterior of a control unit 12' to vary the pressure of the atomizing gas supply to notch the requirements of the particular nebulizer 10 being used. This allows use of different sizes of nebulizers with the control unit 12'. A pressure gauge 42 is shown connected to the compressed gas line 36 between the pressure regulator 40 and the third valve port 35. The pressure gauge 42 is used by the therapist in setting the gas pressure to be supplied to the nebulizer 10. In the embodiment illustrated, the air compressor 37 can provide pressurized air at, for example, a pressure of 28 PSI which is reduced by the pressure regulator 40 to a desired pressure within the range of 4 to 20 PSI.

Other embodiments involve alternate modes of operation for the nebulizer 10. For example, the control logic module 30 can keep the valve solenoid 31 continuously energized to provide a continuous flow of pressurized gas to the nebulizer 10 for non-metered delivery of atomized medicine to the patient. The control logic module 30 can be designed to rapidly pulse the valve solenoid 31 to cause a pulsating pressurized air flow to the nebulizer 10. This mode of operation increases the medicine atomization rate. Such modes of operation can be selected by the therapist by means of a mode selection switch 45 mounted on the exterior of the control unit 12'. Status indicators, though not shown in the drawings, also can be easily added to the control unit 16 to display the mode of operation and current status of the system.

The system also may have a small accumulator tank 46 connected to the compressed gas line 36 between the air compressor 37 and the pressure regulator 40. The accumulator tank 46 stores a reserve of compressed air that is used should the capacity of the air compressor be exceeded, allowing use of a smaller air compressor.

Various other modifications and changes will be apparent to those skilled in the art without departing from the spirit and the scope of the following claims.

## Claims

1. A control system for a medical nebulizer for treatment of a patient, said nebulizer containing a medication and including first and second inlets and an outlet, said first inlet being open to the atmosphere and said outlet to be used by the patient for respiration, said control system comprising a compressed gas supply, means for sensing gas flow direction, valve means for controlling gas flow having a first valve port connected to said second nebulizer inlet, a second valve port connected to said means for sensing gas flow direction, and a third valve port connected to said compressed gas supply, said valve means having a first position wherein said first valve port is connected to said second valve port and a second position wherein said first valve port is connected to said third valve port, and control means responsive to said gas flow sensing means for moving said valve means from said first position to said second position when the patient begins inhalation.

2. A nebulizer control system as set forth in claim 1, wherein said control means includes a pushbutton switch, and control logic means for causing said valve means to move from said first position to said second position only when said sensing means indicates the patient is inhaling and said pushbutton switch has been depressed.

3. A nebulizer control system as set forth in claim 2, wherein said control logic means includes timer means for causing said valve means to return from said second position to said first position after said valve means is in said second position for a predetermined time.

4. A nebulizer control system as set forth in claim 3, and including means for adjusting said predetermined time.

5. A nebulizer control system as set forth in claim 1, wherein said control system includes a pressure regulator connected between said compressed gas supply and said third valve port of said control valve means.

6. A nebulizer control system as set forth in claim 5, wherein said system includes a pressure gauge connected between said pressure regulator and said third valve port of said control valve means, and wherein said pressure regulator is adjustable.

7. A nebulizer control system as set forth in claim 1, wherein said valve means includes a three way solenoid operated valve.

8. A nebulizer control system as set forth in claim 1, wherein said means for sensing gas flow includes a mass airflow sensor.

9. A nebulizer control system as set forth in claim 1, wherein said compressed gas supply includes an air compressor.

10. A nebulizer control system as set forth in claim 9, wherein said compressed gas supply further includes a gas accumulator tank connected between said air compressor and said pressure regulator for storage of a volume of compressed air.

FIG. 1

FIG. 2

6

FIG. 3

CONTROL
LOGIC
MODULE

AIR
COMPRESSOR

FIG. 4

7

European Patent
Office

**EUROPEAN SEARCH REPORT**

Application Number

EP 92 30 5661

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| Y | DE-A-2 809 255 (ROSENTHAL) <br> * claims 1-5; figures 1-4B * <br> --- | 1-10 | A61M15/00 |
| Y | WO-A-8 903 699 (PERKINS) <br> * claims 1,7; figure 4 * <br> --- | 1-10 | |
| A | US-A-4 570 631 (DURKAN) <br><br> * column 3, line 19 - column 4, line 48 * <br> * column 6, line 25 - line 29; figures 1-3 * <br> --- | 1,3,4,5, 7,10 | |
| A | GB-A-2 164 569 (ETELA-HAMEEN) <br><br> ----- | - | |
| | | | TECHNICAL FIELDS SEARCHED (Int. Cl.5) |
| | | | A61M |

The present search report has been drawn up for all claims

| Place of search | Date of completion of the search | Examiner |
|---|---|---|
| THE HAGUE | 23 SEPTEMBER 1992 | VILLENEUVE J.M. |

EPO FORM 1503 03.82 (P0401)